# EUROPÄISCHE PATENTANMELDUNG

(11) **EP 4 560 006 A1**
(43) Veröffentlichungstag der Anmeldung: **28.05.2025**
(21) Anmeldenummer: 23211254.0
(22) Anmeldetag: 21.11.2023
(51) Int. Cl.: C12N 1/16, A01K 63/00, A01K 63/04, F17C 1/00

(54) **AQUARIENDRUCKGASBEHÄLTER-CO2-ERZEUGUNGSZUSAMMENSETZUNG, AQUARIENDRUCKGASBEHÄLTER-CO2-ERZEUGUNGSZUSAMMENSETZUNGSVERPACKUNG UND AQUARIENDRUCKGASBEHÄLTER**

(71) Anmelder: SunBau GmbH, 40699 Erkrath (DE)
(72) Erfinder: Ksienzyk, Horst Walther, 40699 Erkrath (DE)
(74) Vertreter: Michalski Hüttermann & Partner Patentanwälte mbB

(57) **Zusammenfassung**

Die vorliegende Erfindung betrifft eine Aquariendruckgasbehälter-CO2 Erzeugungszusammensetzung zur Mischung mit Gärungswasser, um CO2-Gas in einem Aquariendruckgasbehälter (10) zu erzeugen, die Aquariendruckgasbehälter-CO2-Erzeugungszusammensetzung aufweisend Dextrose und Hefe zur Gärung von Saccharose und Dextrose.

Des Weiteren betrifft die vorliegende Erfindung eine Aquariendruckgasbehälter-CO2-Erzeugungszusammensetzungsverpackung mit der Aquariendruckgasbehälter-CO2-Erzeugungszusammensetzung.

Des Weiteren betrifft die vorliegende Erfindung einen Aquariendruckgasbehälter, welcher mit der Aquariendruckgasbehälter-CO2-Erzeugungszusammensetzung zu begasen ist.

## Beschreibung

### Technisches Gebiet

Die vorliegende Erfindung betrifft eine Aquariendruckgasbehälter-CO2-Erzeugungszusammensetzung.

Des Weiteren betrifft die vorliegende Erfindung eine Aquariendruckgasbehälter-CO2-Erzeugungszusammensetzungsverpackung mit der Aquariendruckgasbehälter-CO2-Erzeugungszusammensetzung.

Des Weiteren betrifft die vorliegende Erfindung einen Aquariendruckgasbehälter, welcher mit der Aquariendruckgasbehälter-CO2-Erzeugungszusammensetzung zu begasen ist.

### Technischer Hintergrund

Für die Sauerstoffversorgung von Fischen ist eine hinreichende Bepflanzung des Aquariums von hoher Bedeutung. Die Pflanzen benötigen hierzu Kohlenstoffdioxid, CO2, welches die Grundlage für den Sauerstoff bildet. Üblicherweise wird CO2-Gas in der Aquaristik zur Pflanzenversorgung und zur pH-Wert-Regulierung eingesetzt. Hierbei wird gasförmiges CO2 im Aquarienwasser gelöst und steht den Pflanzen für die Photosynthese beziehungsweise zum Zellaufbau zur Verfügung. Zur pH-Wert-Regulierung wird das gasförmige CO2 ebenfalls im Wasser gelöst, sodass dessen pH-Wert sinkt.

Grundsätzlich kann gasförmiges CO2 dem Wasser über eine Mess- und Regelungselektronik zudosiert werden. Hierzu werden in der Aquaristik seit Jahrzehnten folgende zwei unterschiedliche Verfahren zur Bereitstellung von CO2-Gas angewandt.

Einerseits wird CO2-Gas mittels CO2-Druckkartuschen bereitgestellt, bei welchen der Kartuschendruck durch einen Druckminderer soweit reduziert wird, dass das CO2-Gas einem Druckniveau entspricht, um das Aquarium ohne weitere Komplikationen mit CO2, das einen Begasungsdruck aufweist, begasen zu können.

Da ohne Lichteinfall in ein Aquarium der Photosynthese-Prozess der darin befindlichen Pflanzen aussetzt, ist es üblich, dass die Zugabe von CO2-Gas während der Nachtstunden pausiert wird. Dies geschieht bei CO2-Druckkartuschen in der Regel durch ein Unterbrechen des Gasflusses im Leitungsverlauf, und somit der CO2-Gaszufuhr. Dieses Pausieren kann beispielhaft durch ein Magnetventil erfolgen.

Zur pH-Wert-Regulierung mit CO2-Druckkartuschen wird die gleiche Technik angewandt, jedoch wird die CO2-Gaszufuhr nicht im Tag/Nacht-Intervall geschaltet, sondern das Pausieren der CO2-Gaszufuhr wird durch eine Regelungselektronik gesteuert, wobei durch eine Permanentmessung des pH-Wertes die Schaltintervalle bestimmt werden.

Bei der Lagerung, beim Betreiben und beim Transport von CO2-Druckkartuschen sind einschlägige Sicherheitsbestimmungen einzuhalten, wobei beispielhaft sicherzustellen ist, dass nationale Sicherheitsbestimmungen hinsichtlich der benutzten CO2-Druckkartuschen gültig sind.

Zum Befüllen von Mehrweg-CO2-Druckkartuschen sind diese an einen Befüllungsort zu transportieren und nach dem Befüllen wieder abzutransportieren. Auch der Transport der CO2-Druckkartuschen unterliegt sicherheitsrechtlichen Bestimmungen. Eine CO2-Druckkartusche verfügt in der Regel über eine Überdrucksicherung, um einen überhöhten Kartuschendruck zu vermeiden und die CO2-Druckkartusche zu schützen. Üblicherweise löst die Überdrucksicherung bei einem Kartuschendruck von etwa 58 bar aus.

CO2-Druckkartuschen mit beispielsweise über 50 Gramm CO2-Befüllung sind aufgrund von nationalen Sicherheitsbestimmungen oft nicht zentrallagerfähig und somit nicht für einen flächendeckenden Vertrieb mit der Verteilung an Einzelhändler geeignet. Andererseits kann CO2-Gas auf biologischem Wege vor Ort, also beim Endverbraucher, selbst produziert und in die zu versorgenden Aquarien geleitet werden. Hierzu weisen CO2-Gärsysteme üblicherweise Gärbehälter aus Kunststoff auf, die in der Regel mit Saccharose befüllt werden. Durch die Zugabe von Hefe wird ein Gärprozess in Gang gesetzt, bei welchem CO2-Gas freigesetzt wird. Der Vorteil dieses Verfahrens liegt darin, dass keine CO2-Druckkartuschen befüllt und transportiert werden müssen. Der Nachteil liegt darin, dass der Gärprozess unregelmäßig erfolgt und erst nach mehreren Tagen startet, nachdem die Saccharose und die Hefe miteinander in Kontakt getreten sind. Aufgrund dieser Problematik wird das Verfahren hauptsächlich bei weniger anspruchsvollen Aquarien angewandt, bei welchen eine schnell eintretende CO2-Produktion und gleichmäßig erfolgende CO2-Freisetzung nicht erforderlich ist. Dies bedeutet allerdings, dass eine CO2-Fehldosierung, insbesondere CO2-Unterdosierung, droht. Somit ist das vorbekannte Gärverfahren auch für eine zuverlässige pH-Wert-Regulierung ungeeignet.

### Beschreibung der Erfindung

Ausgehend von dieser Situation ist es eine Aufgabe der vorliegenden Erfindung, eine Aquariendruckgasbehälter-CO2-Erzeugungszusammensetzung, eine Aquariendruckgasbehälter-CO2-Erzeugungszusammensetzungsverpackung und einen Aquariendruckgasbehälter zur Verfügung zu stellen, die die vorgenannten Nachteile überwinden.

Insbesondere kann die Aufgabe eine Aquariendruckgasbehälter-CO2-Erzeugungszusammensetzung, eine Aquariendruckgasbehälter-CO2-Erzeugungszusammensetzungsverpackung und einen Aquariendruckgasbehälter betreffen, welche eine schnell eintretende und gleichmäßig erfolgende CO2-Freisetzung ermöglichen.

Die Aufgabe der Erfindung wird durch die Merkmale der unabhängigen Hauptansprüche gelöst. Vorteilhafte Ausgestaltungen sind in den Unteransprüchen angegeben. Sofern technisch möglich, können die Lehren der Unteransprüche beliebig mit den Lehren der Haupt- und Unteransprüche kombiniert werden.

Insbesondere wird die Aufgabe demnach gelöst durch eine Aquariendruckgasbehälter-CO2-Erzeugungszusammensetzung zur Mischung mit Gärungswasser, um CO2-Gas in einem Aquariendruckgasbehälter zu erzeugen, die Aquariendruckgasbehälter-CO2-Erzeugungszusammensetzung aufweisend Dextrose und Hefe zur Gärung von Saccharose und Dextrose.

Nachfolgend werden vorteilige Aspekte der beanspruchten Erfindung erläutert und weiter nachfolgend bevorzugte modifizierte Ausführungsformen der Erfindung beschrieben. Erläuterungen, insbesondere zu Vorteilen und Definitionen von Merkmalen, sind dem Grunde nach beschreibende und bevorzugte, jedoch nicht limitierende Beispiele. Sofern eine Erläuterung limitierend ist, wird dies ausdrücklich erwähnt.

In anderen Worten ist insbesondere vorgesehen, dass die Hefe mit zwei unterschiedlichen Zuckern wechselwirkt. Dabei gibt es Unterschiede zwischen der Gärung von Saccharose und Dextrose durch Hefe. Saccharose und Dextrose sind beides Zuckerarten, aber sie haben unterschiedliche chemische Strukturen, die sich auf die Gärung auswirken.

Gärungswasser kann Leitungswasser, Quellwasser, Tafelwasser, Mineralwasser, artähnliches Wasser oder eine Mischung aus diesen Wassern sein. Es handelt sich um das Wasser, das mit Hefe, Dextrose und Saccharose vermischt wird, sodass der Gärprozess von Dextrose und Saccharose durch die Hefe erfolgt. Das Gärungswasser befindet sich insbesondere in dem Aquariendruckgasbehälter. Um Hefe zu aktivieren, hat das Gärungswasser mit Vorzug eine Temperatur zwischen einschließlich 30 °C und einschließlich 40 °C, mit besonderem Vorzug zwischen einschließlich 32 °C und einschließlich 38 °C. Wenn das Wasser zu kühl ist, wird die Hefe nicht aktiviert. Wenn es zu heiß ist, kann die Hefe absterben. Das Gärungswasser muss kein unmittelbarer Bestandteil der Aquariendruckgasbehälter-CO2-Erzeugungszusammensetzung sein, kann es jedoch sein. Vorteilig ist das Gärungswasser jedoch kein eingemischter Bestandteil, da die Hinzugabe von entsprechendem Gärungswasser die Gärung in Gang setzt.

Hefe ist ein zumeist einzelliger pilzähnlicher Organismus, der sich durch Teilung oder "Sprossung" vermehrt. Sie ist bekannt für ihre Rolle in der Back- und Brauindustrie, wo sie zur Herstellung von Brot, Bier und Wein verwendet wird.

Hefezellen sind klein und werden erst mit einem Mikroskop mit 500 bis 800facher Vergrößerung sichtbar. Die bekannteste Form ist die Bäckerhefe, auch als "Saccharomycetaceae" bezeichnet.

Die vorliegende Hefe ist für den Fachmann verständlich eine aktive Hefe. Insbesondere kann diese vor der Verwendung aktiviert werden. Als beispielhafte Trockenhefe besteht sie häufig aus kleinen, länglichen Körnchen und kann bei Raumtemperatur mit Vorzug mindestens ein Jahr lang gelagert oder beispielhaft für mehr als 10 Jahre eingefroren werden. Aktive Trockenhefe muss in lauwarmem Wasser aufgelöst werden, bevor sie zu den restlichen Zutaten hinzugefügt wird. Dies liegt daran, dass sie ein lebender Organismus ist und solange ruht, bis sie aktiviert wird.

Die Hefe ist ausgewählt bzw. ausgebildet zur Gärung von Saccharose und Dextrose. Insbesondere ist die Hefe separat verpackt von der Dextrose und/oder Saccharose.

Saccharose, auch bekannt als Haushaltszucker, ist ein Disaccharid, das aus einem Glucose- und einem Fructosemolekül besteht. Bevor die Hefe die Saccharose fermentieren kann, muss sie zuerst in ihre Bestandteile Glucose und Fructose zerlegt werden. Dieser zusätzliche Schritt kann dazu führen, dass die Gärung von Saccharose länger dauert. Da es sich um Naturprodukte handelt, ist der genaue Zeitpunkt der CO2-Erzeugung nie genau vorherzusagen, liegt jedoch zwischen zwei bis drei Tagen. In der Aquaristik stellt die Fermentierung von Saccharose durch Hefe bis zum vorliegenden Anmeldezeitpunkt den Standard dar. Die Saccharose kann, muss jedoch kein Bestandteil der Aquariendruckgasbehälter-CO2-Erzeugungszusammensetzung sein.

Dextrose hingegen ist ein anderer Name für Glucose, also Traubenzucker. Sie besteht aus einem Zuckermolekül beziehungsweise Einfachzucker. Da Dextrose bereits ein Einfachzucker ist, kann die Hefe sie direkt fermentieren, ohne dass sie zuerst zerlegt werden muss. Mithin beginnt die Gärung von Dextrose schneller.

Es ist zu beachten, dass die genauen Zeiten für den Beginn der Gärung und die Produktion von CO2 nicht exakt vorhersehbar sind. Auch ist zu beachten, dass die Gärung insbesondere zum Anfang noch starken Schwankungen unterliegt. Es hat sich jedoch herausgestellt, dass die Gärung von Saccharose zum Anfang unregelmäßiger verläuft als die Gärung von Dextrose, jeweils durch Hefe. Dies ist damit zu begründen, dass die Saccharose anfangs noch in ihre Bestandteile Glucose und Fructose zerlegt werden muss. Auch wenn die Hefe dem Grunde nach direkt mit der Gärung beginnt, sind die in dieser Phase die entsprechend nur begrenzt verfügbaren Gärungsmittel unregelmäßig vorhanden, sodass eine entsprechend holprige CO2-Erzeugung stattfindet, was zu einem wechselhaften pH-Wert im Aquarium führt und im Ergebnis den Fischen zwischenzeitlich schadet.

Durch die Zugabe von Dextrose gibt es zwei Gärungsphasen für die Hefe. Zunächst beginnt sie die CO2-erzeugende Gärung der Dextrose. Dieser Gärungsschritt kann vorliegend als Phase-1-Gärung bezeichnet werden. Parallel wird die Saccharose zersetzt. Sobald genügend Saccharose zersetzt wurde, steht beispielsweise auch deren Glucose zur Gärung zur Verfügung, sodass damit eine weitere CO2-erzeugende Gärung erfolgt, welche vorliegend als Phase-2-Gärung bezeichnet werden kann. Allerdings dauert es wegen der noch erfolgenden Saccharosezersetzung, bis eine gleichmäßige Phase-2-Gärung stattfindet. Damit überbrückt die Phase-1-Gärung die Zeit bis genügend Saccharose zersetzt worden ist, um eine stabile Phase-2-Gärung durchzuführen. Für die Aquaristik hat sich dies als besonders vorteilig erwiesen, da hiermit in möglichst kurzer Zeit eine zuverlässige pH-Wert-Regulierung erfolgen kann und die Gesundheit der Fische damit nicht, wie bislang üblich, bis zu einer stabilen Gärung der Saccharose gefährdet beziehungsweise belastet wird.

Wie bereits erwähnt, kann die Saccharose, muss jedoch kein Bestandteil der Aquariendruckgasbehälter-CO2-Erzeugungszusammensetzung sein. Damit kann die Abwesenheit der Saccharose aus der Aquariendruckgasbehälter-CO2-Erzeugungszusammensetzung, insbesondere im Sinne eines Disclaimers, insofern als besonderes Merkmal verstanden werden, als der Verbraucher die Aquariendruckgasbehälter-CO2-Erzeugungszusammensetzung mit der Hefe und der Dextrose unabhängig von der Saccharose erwerben kann. Da die Dextrose nur zur Überbrückung von der Phase-1-Gärung bis zur Phase-2-Gärung dient, und damit die Funktion eines Boosters und nicht die eines Hauptgärungsmittels hat, wird sie in geringerer Menge als die Saccharose benötigt. Der Verbraucher kann somit beispielsweise Saccharose als günstig im Supermarkt verfügbaren Haushaltszucker selbst kaufen und muss diesen nicht beim Aquariendruckgasbehälter-CO2-Erzeugungszusammensetzung mitkaufen. Dies ermöglicht, dass jene Mittel, die für die Phase-1-Gärung von hoher Relevanz sind, um eine schnellen Gärungsprozess zu ermöglichen, in gesicherter Qualität und Mengenanteil bereitgestellt werden können sowie bedarfsweise mit Zusätzen angereichert werden können. Die Saccharose ist für die Langzeit-CO2-Erzeugung vorgesehen und kann vom Verbraucher in der entsprechenden Menge frei ausgewählt und hinzugefügt werden. Wenn die Saccharose Bestandteil der Aquariendruckgasbehälter-CO2-Erzeugungszusammensetzung ist, ist die Saccharose insbesondere in der notwendigen Qualität und Mengenanteil beigefügt.

Gemäß einer Modifikation ist die Hefe eine Trockenhefe, die eine aktive Trockenhefe oder eine Instant-Hefe ist. Trockenhefe ist grundsätzlich eine aktive Hefe, die zum Gärprozess zu aktivieren ist, da die Hefezellen durch den Trocknungsprozess in einen Ruhezustand versetzt werden. Die in der Trockenhefe enthaltenen Pilzkulturen liegen ruhend vor und werden erst durch Flüssigkeitszugabe aktiviert. Trotzdem handelt es sich um lebende Mikroorganismen - nur in einem "Ruhezustand". Es ist zu beachten, dass es verschiedene Arten von Trockenhefe gibt, beispielhaft Instant- und Aktiv-Trockenhefe. Die beispielhaft erwähnte Instant-Hefe muss nicht aktiviert werden: sie ist mit den trockenen Zutaten zu vermischen. Bei der beispielhaft erwähnten aktiven Trockenhefe ist die Hefe insbesondere zuerst zu aktivieren, beispielsweise durch Hinzugabe von lauwarmem Wasser.

Gemäß einer Modifikation ist die Hefe Saccharomyces Bayanus, insbesondere Lalvin EC 1118. Saccharomyces Bayanus hat eine für Hefen hohe Alkoholtoleranz. So kann Saccharomyces Bayanus Alkoholkonzentrationen von bis zu 18 Volumen-% tolerieren. Dies erlaubt eine möglichst lange Gärung, wodurch sich die erforderliche Wechselfrequenz der Aquariendruckgasbehälter-CO2-Erzeugungszusammensetzung zum Wohle der im Aquarium befindlichen Lebewesen reduziert. Lalvin EC 1118 ist ein speziell selektionierter Trockenreinzuchthefestamm von Saccharomyces Bayanus, der sehr zuverlässig bei der Vergärung einsetzbar ist. Es zeigt einen günstigen Gärverlauf mit hohem Endvergärungsgrad. Wildhefen und unerwünschte Bakterien werden unterdrückt. Lalvin EC 1118 Hefe erzeugt keine unerwünschten Gärnebenprodukte wie SO2, H2S, Acetaldehyd, Brenztraubensäure (Pyruvat), α-Ketoglutarsäure, flüchtige Säure und Ester. Mithin reguliert die in der Aquaristik gänzlich unübliche Hefe Saccharomyces Bayanus, insbesondere Lalvin EC 1118, wegen der stabilen und lang anhaltenden Gärprozesse den pH-Wert im Aquarium möglichst robust und reduziert den Stress auf im Aquarium befindliche Lebewesen.

Gemäß einer Modifikation weist die Hefe einen Hefestamm auf, der bis zu im Wesentlichen mindestens einschließlich 15,8 Volumen-%, bevorzugt bis zu im Wesentlichen mindestens einschließlich 18 Volumen-% alkoholtolerant ist. Die Formulierung im Wesentlichen umfasst hierbei eine Abweichung von 10 % nach oben bzw. unten vom angegebenen Wert. Dies ermöglicht lang anhaltende Gärprozesse, hält den pH-Wert im Aquarium möglichst robust und reduziert den Stress auf im Aquarium befindliche Lebewesen.

Gemäß einer Modifikation weist die Hefe einen oder mehrere Zusätze auf. Diese können, wie nachfolgend beispielhaft erwähnt, diverse Effekte haben. Mit besonderem Vorzug, und nicht abschließend, ermöglichen Zusätze gegenüber reinen Hefesorten eine stabilere, längere, schneller eintretende und effizientere Gärung.

Gemäß einer Modifikation weist die Hefe als mindestens einen Zusatz ein oder mehrere Spurenelemente auf, insbesondere mindestens eines oder mehrere Spurenelemente aus den nachfolgenden: Eisen, Zink, Jod, Selen, Kupfer, Mangan, Chrom, Molybdän, Nickel und/oder Kobalt.

Beispiele für Spurenelemente, die in der Hefegärung eine Rolle spielen, sind die vorgenannten Spurenelemente Eisen, Zink, Jod, Selen, Kupfer, Mangan, Chrom, Molybdän, Nickel und/oder Kobalt. Diese werden von den Hefen als Co-Faktoren bei enzymatischen Reaktionen verwendet. Sie sind wichtig für den Stoffwechsel der Hefe und tragen zur allgemeinen Gesundheit und Vitalität der Hefezellen bei.

Eisen ist ein wichtiger Kofaktor für viele Enzyme in Hefezellen und spielt eine entscheidende Rolle bei Prozessen wie der DNA-Synthese und dem Energiestoffwechsel. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist. Zink ist ein essentielles Spurenelement für Hefen und ist an einer Vielzahl von zellulären Funktionen beteiligt, einschließlich dem Wachstum. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist.

Jod ist nicht als essentielles Element für Hefen bekannt, aber es hat sich herausgestellt, dass es bezüglich eines kontrollierten Wachstums der Hefe zuträglich ist. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist.

Selen ist ein wichtiger Bestandteil einiger Enzyme und stabilisiert das Wachstum und die Überlebensfähigkeit von Hefen. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist.

Kupfer ist ein wichtiger Kofaktor für viele Enzyme in Hefen und ist an Prozessen wie dem oxidativen Stressschutz und dem Eisenstoffwechsel beteiligt. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist.

Mangan ist ein wichtiger Kofaktor für eine Reihe von Enzymen in Hefen und spielt eine Rolle bei Prozessen wie dem oxidativen Stressschutz und dem Metabolismus von Aminosäuren und Kohlenhydraten. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist.

Chrom ermöglicht überraschenderweise einen robusten Zuckerstoffwechsel. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist.

Molybdän ist ein wichtiger Kofaktor für eine Reihe von Enzymen in Hefen und spielt eine Rolle bei Prozessen wie dem Stickstoffstoffwechsel und dem Schutz vor oxidativem Stress. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist.

Nickelspielt überraschenderweise eine Rolle bei Prozessen wie der Enzymaktivität und dem Schutz vor oxidativem Stress. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist.

Kobalt ist ein Bestandteil von Vitamin B 12, das von einigen Hefen verwendet wird. Es spielt eine Rolle bei Prozessen wie der DNA-Synthese und dem Metabolismus von Fettsäuren und Aminosäuren. Hierbei kann es sich um das einzige Spurenelement handeln. Es ist jedoch auch möglich, dass dieses Spurenelement mit einem oder mehreren der weiteren Spurenelemente kombiniert ist. Ebenso kann es sein, dass dieses Spurenelement nicht vorhanden ist.

Gemäß einer Modifikation weist die Hefe als mindestens einen Zusatz ein oder mehrere Mineralien auf, insbesondere mindestens eines oder mehrere Mineralien aus den nachfolgenden: Kalium, Magnesium, Phosphor und/oder Schwefel.

Mineralien wie Kalium, Magnesium, Phosphor und Schwefel sind vorzugswürdig für die robuste Hefegärung. Sie spielen eine Rolle bei verschiedenen Stoffwechselprozessen und tragen zur allgemeinen Gesundheit und Vitalität der Hefezellen bei.

Kalium ist ein wichtiger Nährstoff für Hefen und spielt eine Rolle bei der Aufrechterhaltung des osmotischen Drucks und der Elektrolythomöostase. Es ist auch an der Aktivierung von Enzymen beteiligt. Hierbei kann es sich um das einzige Mineral handeln. Es ist jedoch auch möglich, dass dieses Mineral mit einem oder mehreren der weiteren Mineral kombiniert ist. Ebenso kann es sein, dass dieses Mineral nicht vorhanden ist.

Magnesium ist ein essentielles Element für Hefen und ist an einer Vielzahl von zellulären Funktionen beteiligt, einschließlich dem Wachstum. Es ist ein wichtiger Kofaktor für viele Enzyme in Hefezellen und spielt eine entscheidende Rolle bei Prozessen wie der DNA-Synthese und dem Energiestoffwechsel. Hierbei kann es sich um das einzige Mineral handeln. Es ist jedoch auch möglich, dass dieses Mineral mit einem oder mehreren der weiteren Mineral kombiniert ist. Ebenso kann es sein, dass dieses Mineral nicht vorhanden ist.

Phosphor ist ein wichtiger Bestandteil von ATP, der Hauptenergiequelle für viele zelluläre Prozesse. In Hefen könnte es eine Rolle bei der DNA-Synthese und der zellulären Energieversorgung spielen. Hierbei kann es sich um das einzige Mineral handeln. Es ist jedoch auch möglich, dass dieses Mineral mit einem oder mehreren der weiteren Mineral kombiniert ist. Ebenso kann es sein, dass dieses Mineral nicht vorhanden ist.

Schwefel ist ein wichtiger Bestandteil einiger Aminosäuren und Proteine. Während der alkoholischen Gärung produzieren Hefen natürlicherweise Schwefeldioxid (SO2) als einen Stoffwechselmittler des Sulfatreduktionsmediums. Hierbei kann es sich um das einzige Mineral handeln. Es ist jedoch auch möglich, dass dieses Mineral mit einem oder mehreren der weiteren Mineral kombiniert ist. Ebenso kann es sein, dass dieses Mineral nicht vorhanden ist.

Gemäß einer Modifikation weist die Hefe als mindestens einen Zusatz Diammoniumphosphat auf.

Diammoniumphosphat (DAP) ist eine anorganische Stickstoffquelle, die von der Hefe leicht aufgenommen werden kann. Es liefert sowohl Stickstoff als auch Phosphor, zwei essentielle Nährstoffe für das Wachstum und die Aktivität der Hefe. Stickstoff ist ein wichtiger Bestandteil von Aminosäuren und Proteinen, während Phosphor eine Schlüsselrolle im Energiestoffwechsel der Hefe spielt.

Stickstoff ist nach Glukose und Fruktose der quantitativ wichtigste Ernährungsfaktor für die Hefe. Es wird zur Synthese verschiedener Moleküle verwendet, hauptsächlich der Proteine. Eine gute Verfügbarkeit von Stickstoff regt die Vermehrung von Hefen an und hält den Gärungsmetabolismus leistungsfähig. Insbesondere kann der Eintritt der Phase-1-Gärung und/oder der Phase-2-Gärung beschleunigt werden.

Phosphor ist ein wichtiger Bestandteil von ATP, der Hauptenergiequelle für viele zelluläre Prozesse. In Hefen könnte es eine Rolle bei der DNA-Synthese und der zellulären Energieversorgung spielen.

Die Zugabe von DAP kann dazu beitragen, die Gärung konstanter zu gestalten und natürliche Schwankungen zu reduzieren, indem sie sicherstellt, dass die Hefe immer Zugang zu diesen wichtigen Nährstoffen hat. Dies kann besonders wichtig sein, wenn das Wasser, in dem die Hefe gärt, nicht genügend natürlichen Stickstoff oder Phosphor enthält.

Gemäß einer Modifikation weist die Hefe als mindestens einen Zusatz ein oder mehrere Hefeautolysate auf, insbesondere mindestens eines oder mehrere Hefeautolysate aus den nachfolgenden: Marmite und/oder Vegemite.

Hefeautolysate, wie sie in Marmite und Vegemite vorkommen, sind reich an Nährstoffen, einschließlich Aminosäuren und Vitaminen. In einer Hefegärung, wie sie in CO2-Behältern für die Aquaristik verwendet wird, können diese Autolysate als Nährstoffquelle dienen, die das Wachstum und die Aktivität der Hefe unterstützen. Sie können auch dazu beitragen, den Gärprozess konstanter zu gestalten und natürliche Schwankungen zu reduzieren, indem sie sicherstellen, dass die Hefe immer Zugang zu diesen wichtigen Nährstoffen hat.

In der Aquaristik bietet sich die Hefegärung an, um CO2 für Pflanzen im Aquarium zu erzeugen. Die Hefe verbraucht Saccharose und/oder Dextrose und produziert dabei Alkohol und CO2. Das CO2 wird dann in das Aquarium abgegeben, wo es von den Pflanzen für die Photosynthese verwendet wird. Hefeautolysate können dazu beitragen, diesen Prozess effizienter und konstanter zu gestalten.

Gemäß einer Modifikation weist die Hefe als mindestens einen Zusatz ein oder mehrere Vitamine auf, insbesondere Vitamin B1.

Vitamin B1, auch Thiamin genannt, ist ein essentielles Vitamin, das eine Schlüsselrolle im Kohlenhydratstoffwechsel der Hefe spielt. Es ist ein Coenzym für mehrere Enzyme, die an der Umwandlung von Saccharose und/oder Dextrose in Energie beteiligt sind. Ein Mangel an Thiamin reduziert die Gärqualität, da die Hefe Saccharose und/oder Dextrose weniger effizient verarbeitet.

In einer Hefemischung, wie sie in CO2-Behältern für die Aquaristik verwendet wird, kann die Zugabe von Vitamin B 1 dazu beitragen, den Gärprozess effizienter und konstanter zu gestalten. Es kann sicherstellen, dass die Hefe stets Zugang zu diesem wichtigen Coenzym hat, das sie für den Zuckerstoffwechsel benötigt.

Gemäß einer Modifikation weist die Hefe als mindestens einen Zusatz inaktive Hefe auf, insbesondere als Trockenhefe.

Inaktive Hefe ist abgetötet und kann keine enzymatische Aktivität mehr entwickeln. Sie kann daher nicht mehr wie die aktive Hefe als Gärungsmittel verwendet werden.

Die alkoholische Gärung läuft im Cytosol von Hefen oder auch bei manchen Bakterien mithilfe von Hefeenzymen ab. Inaktive Hefe kann in diesem Prozess als Nährstoffquelle für die aktive Hefe dienen, da sie reich an Vitaminen und Mineralien ist. Hierdurch erfolgt eine robustere Gärung und die zwischenzeitliche Ausfallwahrscheinlichkeit des Gärungsprozesses wird reduziert.

Gemäß einer Modifikation weist die Hefe mit einem oder mehreren der oben genannten Zusätze ein Mischverhältnis von im Wesentlichen 1 zu 1 auf. Im Wesentlichen bedeutet hierbei eine Abweichung von 10 % nach oben bzw. nach unten des jeweiligen Werts. Wird also eine Hefe mit einem Gramm gewählt, so beträgt der Zusatz ebenfalls ein Gramm. Es hat sich herausgestellt, dass dieser außergewöhnlich hohe Zusatzanteil am Mischverhältnis eine sehr robuste und schnell eintretende Gärung ermöglicht. Entgegen der allgemeinen Annahme, dass die Gärung aufgrund des reduzierten Hefeanteils und des erhöhten Zusatzstoffanteils früher endet, als wenn die gesamte Menge reine Hefe wäre, ist eine längere Gärung möglich, da alle Zusatzstoffe eine effizientere Gärung ermöglichen, entweder weil sie die Gärung optimieren oder weil sie selbst als Hefenahrung dienen.

Gemäß einer Modifikation sind zwei, drei, vier oder mehr, insbesondere alle, Bestandteile der Aquariendruckgasbehälter-CO2-Erzeugungszusammensetzung abhängig vom Aquarienwasservolumen des zu begasenden Aquariums linear skalierbar. Mögliche Bestandteile sind die Hefe, ihre Zusätze, Gärungswasser, Dextrose und/oder Saccharose. Es können jedoch auch andere Bestandteile inbegriffen sein. Bevorzugt sind die Bestandteile einzeln abgepackt, besonders bevorzugt in Mindestmengen, die eine entsprechende Skalierbarkeit ermöglichen, wobei insbesondere gemäß den nachfolgenden Ausführungen das Aquarienwasservolumen als Basiswert herangezogen werden kann.

Gemäß einer Modifikation weist die Aquariendruckgasbehälter-CO2-Erzeugungszusammensetzung Saccharose und/oder Gärungswasser auf. Saccharose ist als Haushaltszucker günstig zu beziehen und daher eine günstige Hefenahrung für einen langen Gärprozess. Die spät einsetzende Phase-2-Gärung wird durch die vorzeitige Dextrose-Gärung während der Phase-1-Gärung ausgeglichen. Gärungswasser kann Leitungswasser, Quellwasser, Tafelwasser, Mineralwasser, artähnliches Wasser oder eine Mischung aus diesen Wassern sein. Mit Vorzug sollte es gärungsfreundlich und leicht verfügbar sein.

Gemäß einer Modifikation beträgt für ein Aquarienwasservolumen des zu begasenden Aquariums von zwischen einschließlich 100 Liter und 150 Liter das Mischverhältnis von Hefe, insbesondere einschließlich einem oder mehreren vorgenannten Zusätzen, zu Saccharose zu Dextrose:
zwischen einschließlich 0,75 Gramm bis einschließlich 1,25 Gramm zu zwischen einschließlich 225 Gramm bis einschließlich 275 Gramm zu zwischen einschließlich 2,35 Gramm bis einschließlich 2,85 Gramm,
insbesondere für ein Aquarienwasservolumen des zu begasenden Aquariums (12) von 125 Litern 1,00 Gramm zu 250 Gramm zu 2,60 Gramm
wobei bevorzugt das Mischverhältnis ausgestaltet ist für ein Volumen eines Gärungswassers von zwischen einschließlich 0,75 Liter bis einschließlich 1,25 Liter, besonders bevorzugt für ein Volumen des Gärungswassers von 1,00 Liter.
Die Angaben von besonders bevorzugten Angaben haben eine optimale Gärung für einen Kalendermonat ergeben. Dies bedeutet für ein einmonatig zu begasendes Aquarium mit einem Aquarienwasservolumen von 125 Litern 1,00 Gramm Hefe inklusive Zusätzen, 250 Gramm Saccharose und 2,60 Gramm Dextrose bei einem Liter Gärungswasser. Insbesondere teilt sich 1,00 Gramm Hefe einschließlich der Zusätze in 0,5 Gramm reine Hefe und 0,5 Gramm der in dieser Offenbarung genannten Zusätze auf.

Gemäß einer Modifikation sind die Hefe, die Dextrose und/oder die Saccharose derart abgepackt sind, dass sie in einem bevorzugten Mischverhältnis, insbesondere wie vorstehend beschrieben, zueinander abgepackt sind. Es können somit isolierte 1,00 Gramm Hefe-Packungen, 250 Gramm Saccharose-Packungen und 2,60 Gramm Dextrose-Packungen vorhanden sein. Diese können für ein einmonatig zu begasendes Aquarium mit einem Aquarienwasservolumen von 125 Litern ausreichen. Mit jeweils zwei isolierten 1,00 Gramm Hefe-Packungen, zwei isolierten 250 Gramm Saccharose-Packungen und zwei isolierten 2,60 Gramm Dextrose-Packungen kann man ein einmonatig zu begasendes Aquarium mit einem Aquarienwasservolumen von 125 Litern über zwei Monate aufeinanderfolgend begasen oder man kann ein einmonatig zu begasendes Aquarium mit einem Aquarienwasservolumen von 250 Litern begasen. Es ist zu beachten, dass die Mischverhältnisse variieren können und die Angaben in diesem Beispiel nur bevorzugte Angaben sind.

Weiterhin ist vorgesehen eine Aquariendruckgasbehälter-CO2-Erzeugungszusammensetzungsverpackung,
wobei die Aquariendruckgasbehälter-CO2-Erzeugungszusammensetzungsverpackung mindestens eine vorgenannte Aquariendruckgasbehälter-CO2-Erzeugungszusammensetzung aufweist.
Insbesondere sind die Hefe einschließlich ihrer Zusätze, die Dextrose und/oder die Saccharose wie vorbeschrieben in Packungen abgepackt. Mit besonderem Vorzug sind nur die Hefe einschließlich ihrer Zusätze und die Dextrose abgepackt. Beispielhaft kann eine Aquariendruckgasbehälter-CO2-Erzeugungszusammensetzungsverpackung vier isolierte 1,00 Gramm Hefe-Packungen mit der Hefe einschließlich ihrer Zusätze sowie vier isolierte 2,60 Gramm Dextrose-Packungen aufweisen. Das beispielhafte jeweils 1 Liter Gärungswasser und/oder die vier isolierten 250 Gramm Saccharose-Packungen können Bestandteil der Aquariendruckgasbehälter-CO2-Erzeugungszusammensetzungsverpackung sein. Optional ist es jedoch auch möglich, dass der Benutzer als Gärungswasser eigenes Leitungswasser bezieht und als Saccharose handelsüblichen Haushaltszucker verwendet.

Es ist zu beachten, dass die Mischverhältnisse variieren können und die Angaben in diesem Beispiel nur bevorzugte Angaben sind.

Gemäß einer Modifikation ist die Aquariendruckgasbehälter-CO2-Erzeugungszusammensetzungsverpackung ausgebildet zur gezielt dosierten Aufnahme von streufähiger Saccharose, welche in ihrer Dosierung einem ganzzahligen Vielfachen des gewünschten Mischverhältnisses, insbesondere nach den oben genannten Mischverhältnissen, entspricht. Die streufähige Saccharose ist insbesondere handelsüblicher Haushaltszucker. Mit besonderem Vorzug sind nur die Hefe einschließlich ihrer Zusätze und die Dextrose abgepackt. Beispielhaft kann eine Aquariendruckgasbehälter-CO2-Erzeugungszusammensetzungsverpackung vier isolierte 1,00 Gramm Hefe-Packungen mit der Hefe einschließlich ihrer Zusätze sowie vier isolierte 2,60 Gramm Dextrose-Packungen aufweisen. Anstelle von jeweils 1 Liter Gärungswasser und vier isolierten 250 Gramm Saccharose-Packungen, kann der Benutzer als Gärungswasser eigenes Leitungswasser beziehen. Als Saccharose kann der Benutzer handelsüblichen Haushaltszucker verwenden, wobei er beispielsweise die Aquariendruckgasbehälter-CO2-Erzeugungszusammensetzungsverpackung, beispielsweise äußerlich als Papp- oder Kunststoffschachtel ausgebildet, entleert und vollständig mit Haushaltszucker befüllt. Die Aquariendruckgasbehälter-CO2-Erzeugungszusammensetzungsverpackung ist derart ausgebildet, dass sie genau 250 Gramm Streu-Haushaltszucker fasst, sodass die Verpackung als ein entsprechendes Maß herangezogen werden kann.

Weiter vorgesehen ist ein Aquariendruckgasbehälter mit einer vorbeschriebenen Aquariendruckgasbehälter-CO2-Erzeugungszusammensetzung und/oder mit einer vorbeschriebenen Aquariendruckgasbehälter-CO2-Erzeugungszusammensetzungsverpackung.

Insbesondere hinsichtlich des Aquariendruckgasbehälters, aber auch hinsichtlich der weiteren Angaben, wird auf die Offenbarungsgehalte der japanischen Patentanmeldung mit dem amtlichen Aktenzeichen 2023-176124 sowie auf die europäische Patentanmeldung mit dem amtlichen Aktenzeichen 20821167.2 verwiesen, welche hiermit beide einbezogen werden.

### Kurze Beschreibung der Zeichnungen

Nachfolgend wird die Erfindung unter Bezugnahme auf die anliegenden Zeichnungen anhand bevorzugter Ausführungsbeispiele näher erläutert. Die Formulierung Figur ist in den Zeichnungen mit Fig. abgekürzt.

In den Zeichnungen zeigen
- Fig. 1: eine schematische Seitenansicht eines CO2-Gärsystems mit einem Gärbehälter zur CO2-Begasung eines Aquariums gemäß einem ersten bevorzugten Ausführungsbeispiel;
- Fig. 2: eine schematische Seitenansicht eines CO2-Gärsystems mit einem Gärbehälter zur CO2-Begasung eines Aquariums gemäß einem zweiten bevorzugten Ausführungsbeispiel ;
- Fig. 3: eine schematische Seitenansicht eines Aquariendruckgasbehälter gemäß einem dritten bevorzugten Ausführungsbeispiel;
- Fig. 4: eine alternative schematische Seitenansicht des Aquariendruckgasbehälters gemäß Figur 3; und
- Fig. 5: eine schematische Draufsicht auf den Aquariendruckgasbehälters gemäß den Figuren 3 und 4.

### Detaillierte Beschreibung der Ausführungsbespiele

Die beschriebenen Ausführungsbeispiele sind lediglich Beispiele, die im Rahmen der Ansprüche auf vielfältige Weise modifiziert und/oder ergänzt werden können. Jedes Merkmal, das für ein bestimmtes Ausführungsbeispiel beschrieben wird, kann eigenständig oder in Kombination mit anderen Merkmalen in einem beliebigen anderen Ausführungsbeispiel genutzt werden. Jedes Merkmal, das für ein Ausführungsbeispiel einer bestimmten Anspruchskategorie beschrieben wird, kann auch in entsprechender Weise in einem Ausführungsbeispiel einer anderen Anspruchskategorie eingesetzt werden.

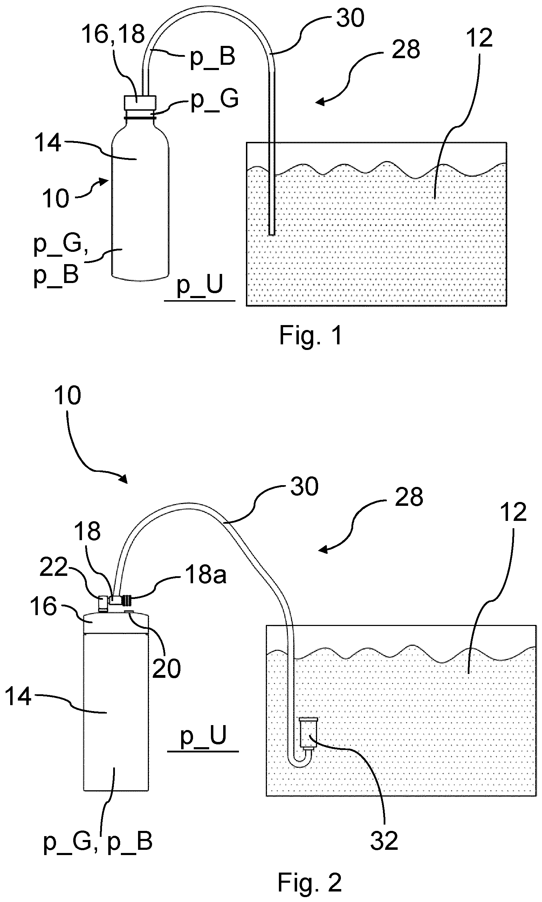

Figur 1 zeigt ein erstes bevorzugtes Gärsystem als CO2-Begasungssystem 28.

Figur 2 zeigt ein alternatives CO2-Begasungssystem 28 gemäß zweiten bevorzugten Ausführungsbeispiel der Erfindung. Dabei weist das CO2-Begasungssystem 28 nach Figur 2 einen Aquariendruckgasbehälter 10 nach einer alternativen bevorzugten Ausführungsform auf.

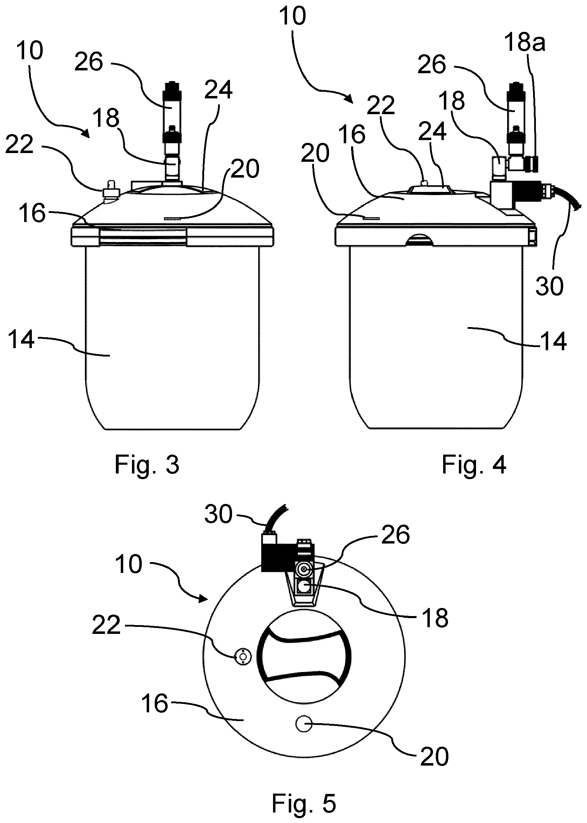

Die Figuren 3 bis 5 zeigen einen Aquariendruckgasbehälter 10 nach einer weiteren alternativen, bevorzugten Ausführungsform, wobei dieser Aquariendruckgasbehälter 10 bevorzugt auch beim CO2-Begasungssystem 28 nach Figur 2 genutzt werden kann. Die Figuren 3 und 4 zeigen den Aquariendruckgasbehälter 10 derselben Ausführungsform in zwei unterschiedlich gedrehten Positionen in einer Seitenansicht. Die Figur 5 offenbart den Aquariendruckgasbehälter 10 nach derselben Ausführungsform in einer Draufsicht.

Figur 1 zeigt ein als Gärsystem ausgebildetes CO2-Begasungssystem 28 mit einem Gärbehälter als Aquariendruckgasbehälter 10 zur Versorgung eines Aquariums 12 mit im Aquariendruckgasbehälter 10 erzeugtem CO2-Gas.

Das Gärsystem umfasst ein Aufnahmegefäß 14 zur Aufnahme einer Aquariendruckgasbehälter-CO2-Erzeugungszusammensetzung mit Hefe, etwaigen Zusätzen, Saccharose, Dextrose und Gärungswasser derart, dass diese Bestandteile miteinander reagieren, um CO2-Gas zu erzeugen. Da das CO2-Gas jedoch sofort ausströmt, baut sich kein Gasbehälterdruck p_G im Gärbehälter auf.

Weiterhin umfasst das Gärsystem eine Verschließeinrichtung 16 zum Verschließen des Aufnahmegefäßes 14.

Die Verschließeinrichtung 16 ist weiterhin als Gasausströmeinrichtung 18 ausgebildet zur CO2-Gas-Entnahme aus dem Gärbehälter für das Aquarium 12, wobei das CO2-Gas beim Einströmen in die Gasausströmeinrichtung 18 einen Begasungsdruck p_B aufweist, der dem Gasbehälterdruck p_G entspricht.

Das Gärsystem weist keinen Absperrmechanismus auf, da dieser zu einem Bersten des Gärbehälters führen könnte.

Das Gärsystem umfasst eine an der Gasausströmeinrichtung 18 angeschlossene CO2-Begasungsleitung 30 zur Versorgung des Aquariums 12 mit dem im Aquariendruckgasbehälter 10 erzeugten CO2-Gas, wobei die CO2-Begasungsleitung 30 ausgebildet ist, um an ihrem von der Gasausströmeinrichtung 18 entfernten Ende in das Aquarium 12 eingetaucht zu sein.

Figur 2 zeigt ein bevorzugtes Ausführungsbeispiel eines CO2-Begasungssystems 28 mit einem beispielhaften Aquariendruckgasbehälter 10.

Der Aquariendruckgasbehälter 10 ist zur Versorgung eines Aquariums 12 mit im Aquariendruckgasbehälter 10 erzeugtem CO2-Gas ausgebildet.

Hierzu weist der Aquariendruckgasbehälter 10 auf:
- ein Aufnahmegefäß 14 zur Aufnahme einer Aquariendruckgasbehälter-CO2-Erzeugungszusammensetzung mit Hefe, etwaigen Zusätzen, Saccharose, Dextrose und Gärungswasser derart, dass diese Bestandteile miteinander reagieren, um CO2-Gas zu erzeugen;
- eine Verschließeinrichtung 16 zum druckdichten Verschließen des Aufnahmegefäßes 14;
- eine Gasausströmeinrichtung 18 zur CO2-Gas-Entnahme aus dem Aquariendruckgasbehälter 10 für das Aquarium 12, wobei das CO2-Gas beim Einströmen in die Gasausströmeinrichtung 18 einen Begasungsdruck p_B aufweist;
- einen Gasbehälterdruckregler 20 zum Einstellen eines Gasbehälterdrucks p_G, der dem Begasungsdruck p_B im Wesentlichen entspricht.

Das CO2-Begasungssystem 28 umfasst weiterhin eine an der Gasausströmeinrichtung 18 angeschlossene CO2-Begasungsleitung 30 zur Versorgung des Aquariums 12 mit dem im Aquariendruckgasbehälter 10 erzeugten CO2-Gas, wobei die CO2-Begasungsleitung 30 ausgebildet ist, um an ihrem von der Gasausströmeinrichtung 18 entfernten Ende in das Aquarium 12 eingetaucht zu sein.

Bevorzugt weist die CO2-Begasungsleitung 30 an ihrem von der Gasausströmeinrichtung 18 entfernten Ende einen CO2-Diffusor 32 auf, wobei der CO2-Diffusor 32 besonders bevorzugt mit einem Durchströmungswiderstand von einschließlich 0,3 bar bis einschließlich 0,6 bar als Überdruck gegenüber einem Umgebungsdruck p_U betreibbar ist.

Weiterhin bevorzugt, jedoch nicht genauer vorgestellt ist, dass der Gasbehälterdruckregler 20 derart ausgebildet ist, dass der Gasbehälterdruck p_G einen Überdruck gegenüber einem Umgebungsdruck p_U von mindestens einschließlich 0,3 bar, bevorzugt mindestens einschließlich 0,5 bar, besonders bevorzugt mindestens einschließlich 0,6 bar beträgt.

Weiterhin bevorzugt, jedoch nicht genauer vorgestellt ist, dass der Gasbehälterdruckregler 20 derart ausgebildet ist, dass der Gasbehälterdruck p_G höchstens einschließlich 3,0 bar, bevorzugt höchstens einschließlich 1,0 bar, besonders bevorzugt höchstens einschließlich 0,9 bar beträgt.

Besonders bevorzugt, jedoch nicht dargestellt, ist der Gasbehälterdruckregler 20 derart ausgebildet, dass der Gasbehälterdruck p_G als Überdruck gegenüber einem Umgebungsdruck p_U 0,8 bar beträgt. Anders formuliert, und als übergreifendes Beispiel, liegt der Gasbehälterdruck p_G um 0,8 bar über dem Umgebungsdruck p_U.

Weiterhin bevorzugt, jedoch nicht genauer vorgestellt ist, dass die Gasausströmeinrichtung 18 ein Nadelventil aufweist und/oder der Gasbehälterdruckregler 20 ein Sicherheitsventil aufweist.

Gemäß den Figuren 2 bis 5 ist bevorzugt, dass der Aquariendruckgasbehälter 10 eine Öffnungssicherung 22 aufweist, um im Falle deren Betätigung den Gasbehälterdruck p_G auf den Umgebungsdruck p_U anzupassen.

Gemäß den Figuren 2 bis 5 ist bevorzugt, dass der Aquariendruckgasbehälter 10 wiederverwendbar ist, wobei insbesondere das Aufnahmegefäß 14 mit der Verschließeinrichtung 16 druckdicht wiederverschließbar ist.

Gemäß Figur 2 ist bevorzugt, dass die Verschließeinrichtung 16 als Schraubdeckel ausgebildet ist.

Gemäß den Figuren 3 bis 5 ist bevorzugt, dass die Verschließeinrichtung 16 als Stülpdeckel, vorzugsweise mit einem Drehverschließmechanismus 24, ausgebildet ist.

Gemäß den Figuren 2 bis 5 ist bevorzugt, dass die Gasausströmeinrichtung 18 einen Absperrmechanismus 18a aufweist, um die CO2-Gas-Entnahme zu stoppen.

Gemäß den Figuren 3 bis 5 ist bevorzugt, dass die Gasausströmeinrichtung 18 eine Begasungsblasendetektionsvorrichtung 26 aufweist, um die Menge des in die Gasausströmeinrichtung 18 einströmenden CO2-Gas zu detektieren.

Gemäß den Figuren 2 bis 5 ist bevorzugt, dass die Gasausströmeinrichtung 18 an der Verschließeinrichtung 16 angeordnet ist, sodass das CO2-Gas aus der Verschließeinrichtung 16 in die Gasausströmeinrichtung 18 einströmen kann.

Weiterhin bevorzugt, jedoch nicht genauer vorgestellt ist, dass der Aquariendruckgasbehälter 10 eine Dichtung aufweist, die umfangsgemäß in einem Kopplungsbereich zwischen dem Aufnahmegefäß 14 und der Verschließeinrichtung 16 angeordnet ist, sodass im geschlossenem Zustand des Aquariendruckgasbehälters 10 das druckdichte Verschließen des Aufnahmegefäßes 14 dadurch entsteht, dass der Gasbehälterdruck p_G die Dichtung in den Kopplungsbereich zwischen dem Aufnahmegefäß 14 und der Verschließeinrichtung 16 presst.

Weiterhin bevorzugt, jedoch nicht genauer vorgestellt ist, dass die Gasausströmeinrichtung 18 und der Gasbehälterdruckregler 20 in einem gemeinsamen Modul angeordnet sind, das mit dem Aufnahmegefäß 14 und/oder mit der Verschließeinrichtung 16 verbunden ist.

Gemäß den Figuren 3 bis 5 ist bevorzugt, dass der Gasbehälterdruckregler 20 mit der Verschließeinrichtung 16 verbunden ist.

Das Gasausströmeinrichtung 18 kann den Absperrmechanismus 18a umfassen. Dies ist jedoch eine optionale Ausgestaltung und unabhängig von anderen Merkmalen.

Das Gasausströmeinrichtung 18 kann den Gasbehälterdruckregler 20 umfassen. Dies ist jedoch eine optionale Ausgestaltung und unabhängig von anderen Merkmalen.

Das Gasausströmeinrichtung 18 kann die Öffnungssicherung 22 umfassen. Dies ist jedoch eine optionale Ausgestaltung und unabhängig von anderen Merkmalen.

In Versuchsreihen wurde eine CO2-erzeugende Gärung in den vorgeschlagenen Aquariendruckgasbehältern 10 erfolgreich getestet.

In das jeweilige Aufnahmegefäß 14 wurde für ein mit 125 Litern Aquarienwasservolumen mit CO2 zu begasendes Aquarium 12: 0,5 Gramm reine Hefe Lalvin EC 1118, 0,5 Gramm Zusätze, aufweisend Spurenelemente, Mineralien, Diammoniumphosphat, Hefeautolysate, inaktive sortengleiche Hefe sowie Vitamin B1, 250 Gramm Haushaltszucker als Saccharose, 2,60 Gramm Dextrose und 1,00 Liter 35 °C warmes Leitungswasser als Gärungswasser gefüllt. Nach 7,25 Stunden begann eine CO2 Produktion, welche sich mit der Blasenzählvorrichtung 26 identifizieren ließ. Die CO2 Produktion verlief sehr konstant und hielt einen Kalendermonat an. Die Zusätze wurden zu gleichen Teilen hinzugefügt, wobei Zink und Kupfer als Spurenelemente, Kalium als Mineral und Vegemite als Hefeautolysate verwendet wurden.

Bei Versuchen für ein Aquarium 12 mit einem doppelten Aquarienwasservolumen von insgesamt 250 Litern zeigte sich, dass die obigen Bestandteile skalierbar sind und eine doppelte Menge eine entsprechend robuste CO2 Produktion ermöglichte.

Demgegenüber begann die CO2 Produktion bei 1,00 Gramm Bäckerhefe, 250 Gramm Haushaltszucker als Saccharose und 1,00 Liter 35 °C warmes Leitungswasser als Gärungswasser für ein mit 125 Litern Aquarienwasservolumen mit CO2 zu begasendes Aquarium 12 mit denselben Aquariendruckgasbehältern 10 erst nach 53 Stunden.

### Bezugszeichenliste

- 10: Aquariendruckgasbehälter
- 12: Aquarium
- 14: Aufnahmegefäß
- 16: Verschließeinrichtung
- 18: Gasausströmeinrichtung
- 18a: Absperrmechanismus
- 20: Gasbehälterdruckregler
- 22: Öffnungssicherung
- 24: Drehverschließmechanismus
- 26: Blasenzählvorrichtung
- 28: CO2-Begasungssystem
- 30: CO2-Begasungsleitung
- 32: CO2-Diffusor

- p_B: Begasungsdruck
- p_G: Gasbehälterdruck
- p_U: Umgebungsdruck

## Patentansprüche

1. Aquariendruckgasbehälter-CO2-Erzeugungszusammensetzung zur Mischung mit Gärungswasser, um CO2-Gas in einem Aquariendruckgasbehälter (10) zu erzeugen, die Aquariendruckgasbehälter-CO2-Erzeugungszusammensetzung aufweisend:
- Dextrose,
- Hefe zur Gärung von Saccharose und Dextrose.

2. Aquariendruckgasbehälter-CO2-Erzeugungszusammensetzung nach Anspruch 1,
wobei die Hefe eine Trockenhefe ist, die eine aktive Trockenhefe oder eine Instant-Hefe ist.

3. Aquariendruckgasbehälter-CO2-Erzeugungszusammensetzung nach mindestens einem der Ansprüche 1 oder 2,
wobei die Hefe Saccharomyces Bayanus, insbesondere Lalvin EC 1118, ist.

4. Aquariendruckgasbehälter-CO2-Erzeugungszusammensetzung nach mindestens einem der vorgenannten Ansprüche,
wobei die Hefe einen Hefestamm aufweist, der bis zu im Wesentlichen mindestens einschließlich 15,8 Volumen-%, bevorzugt bis zu im Wesentlichen mindestens einschließlich 18 Volumen-% alkoholtolerant ist.

5. Aquariendruckgasbehälter-CO2-Erzeugungszusammensetzung nach mindestens einem der vorgenannten Ansprüche,
wobei die Hefe einen oder mehrere Zusätze aufweist.

6. Aquariendruckgasbehälter-CO2-Erzeugungszusammensetzung nach Anspruch 5,
wobei die Hefe als mindestens einen Zusatz ein oder mehrere Spurenelemente aufweist, insbesondere mindestens eines oder mehrere Spurenelemente aus den nachfolgenden:
Eisen, Zink, Jod, Selen, Kupfer, Mangan, Chrom, Molybdän, Nickel und/oder Kobalt.

7. Aquariendruckgasbehälter-CO2-Erzeugungszusammensetzung nach mindestens einem der Ansprüche 5 oder 6,
wobei die Hefe als mindestens einen Zusatz ein oder mehrere Mineralien aufweist, insbesondere mindestens eines oder mehrere Mineralien aus den nachfolgenden:
Kalium, Magnesium, Phosphor und/oder Schwefel.

8. Aquariendruckgasbehälter-CO2-Erzeugungszusammensetzung nach mindestens einem der Ansprüche 5 bis 7,
wobei die Hefe als mindestens einen Zusatz Diammoniumphosphat aufweist.

9. Aquariendruckgasbehälter-CO2-Erzeugungszusammensetzung nach mindestens einem der Ansprüche 5 bis 8,
wobei die Hefe als mindestens einen Zusatz ein oder mehrere Hefeautolysate aufweist, insbesondere mindestens eines oder mehrere Hefeautolysate aus den nachfolgenden:
Marmite und/oder Vegemite.

10. Aquariendruckgasbehälter-CO2-Erzeugungszusammensetzung nach mindestens einem der Ansprüche 5 bis 9,
wobei die Hefe als mindestens einen Zusatz ein oder mehrere Vitamine aufweist, insbesondere Vitamin B 1.

11. Aquariendruckgasbehälter-CO2-Erzeugungszusammensetzung nach mindestens einem der Ansprüche 5 bis 9,
wobei die Hefe als mindestens einen Zusatz inaktive Hefe aufweist, insbesondere als Trockenhefe.

12. Aquariendruckgasbehälter-CO2-Erzeugungszusammensetzung nach mindestens einem der vorgenannten Ansprüche,
wobei die Hefe mit einem oder mehreren Zusätze nach den Ansprüchen 5 bis 11 ein Mischverhältnis von im Wesentlichen 1 zu 1 aufweist.

13. Aquariendruckgasbehälter-CO2-Erzeugungszusammensetzung nach mindestens einem der vorgenannten Ansprüche,
wobei zwei, drei, vier oder mehr, insbesondere alle, Bestandteile der Aquariendruckgasbehälter-CO2-Erzeugungszusammensetzung abhängig vom Aquarienwasservolumen des zu begasenden Aquariums (12) linear skalierbar sind.

14. Aquariendruckgasbehälter-CO2-Erzeugungszusammensetzung nach mindestens einem der vorgenannten Ansprüche,
wobei die Aquariendruckgasbehälter-CO2-Erzeugungszusammensetzung Saccharose und/oder Gärungswasser aufweist.

15. Aquariendruckgasbehälter-CO2-Erzeugungszusammensetzung nach mindestens einem der vorgenannten Ansprüche,
wobei für ein Aquarienwasservolumen des zu begasenden Aquariums (12) von zwischen einschließlich 100 Liter und 150 Liter das Mischverhältnis von Hefe, insbesondere einschließlich einem oder mehreren Zusätzen nach einem der Ansprüche 5 bis 11, zu Saccharose zu Dextrose beträgt:
zwischen einschließlich 0,75 Gramm bis einschließlich 1,25 Gramm zu zwischen einschließlich 225 Gramm bis einschließlich 275 Gramm zu zwischen einschließlich 2,35 Gramm bis einschließlich 2,85 Gramm,
insbesondere für ein Aquarienwasservolumen des zu begasenden Aquariums (12) von 125 Litern 1,00 Gramm zu 250 Gramm zu 2,60 Gramm
wobei bevorzugt das Mischverhältnis ausgestaltet ist für ein Volumen eines Gärungswassers von zwischen einschließlich 0,75 Liter bis einschließlich 1,25 Liter, besonders bevorzugt für ein Volumen des Gärungswassers von 1,00 Liter.

16. Aquariendruckgasbehälter-CO2-Erzeugungszusammensetzung nach mindestens einem der vorgenannten Ansprüche,
wobei die Hefe, die Dextrose und/oder die Saccharose derart abgepackt ist/sind, dass sie in einem bevorzugten Mischverhältnis, insbesondere nach Anspruch 15, zueinander abgepackt sind.

17. Aquariendruckgasbehälter-CO2-Erzeugungszusammensetzungsverpackung,
wobei die Aquariendruckgasbehälter-CO2-Erzeugungszusammensetzungsverpackung aufweist mindestens eine Aquariendruckgasbehälter-CO2-Erzeugungszusammensetzung nach mindestens einem der Ansprüche 1 bis 16,
wobei insbesondere die Hefe, die Dextrose und/oder die Saccharose nach Anspruch 16 in jeweils einer oder mehreren Verpackungseinheiten abgepackt ist/sind.

18. Aquariendruckgasbehälter-CO2-Erzeugungszusammensetzungsverpackung nach Anspruch 17,
wobei die Aquariendruckgasbehälter-CO2-Erzeugungszusammensetzungsverpackung ausgebildet ist zur gezielt dosierten Aufnahme von streufähiger Saccharose, welche in ihrer Dosierung einem ganzzahligen Vielfachen des gewünschten Mischverhältnisses, insbesondere nach Anspruch 15, entspricht.

19. Aquariendruckgasbehälter mit einer Aquariendruckgasbehälter-CO2-Erzeugungszusammensetzung nach mindestens einem der Ansprüche 1 bis 16 und/oder einer Aquariendruckgasbehälter-CO2-Erzeugungszusammensetzungsverpackung nach mindestens einem der Ansprüche 17 bis 18.
